# EUROPÄISCHE PATENTANMELDUNG

(11) **EP 4 660 668 A1**
(43) Veröffentlichungstag der Anmeldung: **10.12.2025**
(21) Anmeldenummer: 24180792.4
(22) Anmeldetag: 07.06.2024
(51) Int. Cl.: G01T 1/20, G01T 1/24, G01T 1/29

(54) **ELEKTRONISCHE SCHALTUNG, PHOTONENZÄHLENDER RÖNTGENDETEKTOR, COMPUTERTOMOGRAPHIESYSTEM UND VERFAHREN ZUM ERFASSEN VON KOINZIDENZEREIGNISSEN EINES COMPUTERTOMOGRAPHIESYSTEMS**

(71) Anmelder: Siemens Healthineers AG, 91301 Forchheim (DE)
(72) Erfinder: Göderer, Edgar, 91301 Forchheim (DE); Hosemann, Michael, 91056 Erlangen (DE); Raffelberg, Pascal, 45057 Duisburg (DE)
(74) Vertreter: Siemens Healthineers Patent Attorneys

(57) **Zusammenfassung**

Elektronische Schaltung (10) zum Erfassen von Koinzidenzereignissen eines Computertomographiesystems, welches einen photonenzählenden Röntgendetektor aufweist, der ein Detektorpixelarray enthält, enthält eine erste Erfassungseinheit (1), die dazu eingerichtet ist, ein erstes Auftreffereignissignal zur Verfügung zu stellen. Außerdem enthält die elektronische Schaltung (10) für jedes weitere Detektorpixel (12) wenigstens eines weiteren Detektorpixels (12) des Detektorpixelarrays eine weitere Erfassungseinheit (1), die dazu eingerichtet ist, ein weiteres Auftreffereignissignal zur Verfügung zu stellen. Die elektronische Schaltung (10) enthält eine Logikschaltung (2), die das erste Auftreffereignissignal mit dem wenigstens einen weiteren Auftreffereignissignal vergleicht und abhängig von einem Ergebnis des Vergleichs ein Koinzidenzsignal zur Verfügung stellt. Ein Koinzidenzzähler (3) der elektronischen Schaltung (10) inkrementiert abhängig von dem Koinzidenzsignal einen Koinzidenzzählerstand des Koinzidenzzählers (3). Darüber hinaus enthält die elektronische Schaltung (10) für jedes weitere Detektorpixel (12) des wenigstens einen weiteren Detektorpixels (12) eine Schalteinheit (4), die dazu eingerichtet ist, das jeweilige weitere Auftreffereignissignal von der Logikschaltung (2) zu trennen.

## Beschreibung

Die Erfindung betrifft eine elektronische Schaltung ein Verfahren zum Erfassen von Koinzidenzereignissen eines Computertomographiesystems, einen photonenzählenden Röntgendetektor mit einer solchen elektronischen Schaltung und ein Computertomographiesystem mit einer solchen elektronischen Schaltung. Das Computertomographiesystem enthält einen photonenzählenden Röntgendetektor mit einem Detektorpixelarray.

Während bei konventionellen Detektoren für Computertomographiesysteme (im Folgenden CT-Systeme) einfallende Röntgenphotonen zunächst in optische Photonen umgewandelt werden (Szintillation), wandeln photonenzählende Röntgendetektoren das Röntgenphoton auf der aktiven Fläche direkt in ein elektrisches Signal um (direkte Konversion). Dabei werden positive und negative elektrische Ladungen, die durch eintreffende Röntgenphotonen erzeugt werden, durch elektrische Felder getrennt und die Ladungsmenge, und folglich die Energie des Röntgenphotons, wird durch die Amplitude des elektrischen Signals widergegeben. Durch die hohe Signaldauer bei Szintillationsdetektoren, die im Bereich von Mikrosekunden liegt, überlagern sich aufeinanderfolgende Ereignisse in der Regel. Aus diesem Grund werden diese Detektoren auch als energieintegrierend bezeichnet. Werden stattdessen sehr schnell auslesbare, beispielsweise im Bereich von Nanosekunden, Halbleiterdetektoren (z.B. basierend auf Silizium, Cadmiumtellurid, Cadmiumzinktellurid oder Galliumarsenid) verwendet, können einzelne Photonen gezählt und können, beispielsweise mithilfe eines Komparators, in ein Histogramm sortiert werden. Diese Technik ermöglicht eine spektrale Trennung und damit eine bessere nachträgliche Materialdifferenzierung. Durch das Umgehen der indirekten Konversion wird das elektrische Rauschen im Detektor reduziert, sodass bessere Signal-Rausch-Verhältnisse erreicht werden bzw. die gleichen Signal-Rausch-Verhältnisse bei weniger Dosis.

Röntgendetektoren in CT-Systemen können beispielsweise eine Anzahl an Detektorpixeln von größer 1 Million aufweisen. Darüber hinaus können CT-Systeme gegebenenfalls auch mehrere solcher Röntgendetektoren aufweisen.

Als ein Koinzidenzereignis wird im Zusammenhang mit CT-Systemen ein gleichzeitiges Auftreten von mindestens zwei Zählereignissen bezeichnet, die jeweils an einem entsprechenden Detektorpixel des photonenzählenden Röntgendetektors erkannt werden. Beispielsweise können diese Zählereignisse in nahe beieinanderliegenden Detektorpixeln erkannt werden. Bei einem Auftreffen eines Röntgenphotons auf die Grenzfläche von zwei aneinandergrenzenden Detektorpixeln ist es beispielsweise möglich, dass die Energie des Röntgenphotons an zwei oder mehr Detektorpixeln erkannt wird. Es ist auch möglich, dass durch Auftreffen des Röntgenphotons auf einem Detektorpixel Sekundärphotonen freigesetzt werden, die dann von einem anderen Detektorpixel erkannt werden. Dies kann zu Bildfehlern des rekonstruierten Bildes führen. Zur Vermeidung oder Reduzierung solcher Fehler können photonenzählende CT-Systeme elektronische Schaltungen zur Erkennung der Koinzidenzereignisse enthalten.

Diese elektronischen Schaltungen können beispielsweise erkennen, ob gleichzeitig Auftreffereignisse in dem Detektorpixel und wenigstens einem weiteren Detektorpixel in einer definierten Nachbarschaft auftreten. Dazu werden beispielsweise Signalleitungen, im Folgenden auch als Koinzidenzleitungen bezeichnet, zwischen den involvierten Detektorpixeln angelegt. Über diese Koinzidenzleitungen kann dann jeweils die Information des Auftreffens bei einem Detektorpixel an die entsprechenden restlichen involvierten Detektorpixel übermittelt werden. Berücksichtigt man beispielsweise Koinzidenzereignisse aus einer Vierer-Nachbarschaft von Detektorpixel, können durch diese Leitungsführung acht Koinzidenzleitungen pro Detektorpixel resultieren. Bei anderen Lösungen können gegebenenfalls noch mehr Koinzidenzleitungen erforderlich sein.

Leitungen zur Übertragung von digitalen Signalen in Form von Pulsen können parasitäre Widerstände und parasitäre Kapazitäten aufweisen. Leitungen können zwischen Adern derselben Leitung und/oder zwischen den Adern und der Leitungsumgebung Anordnungen bilden, die ähnlich einem Kondensator wirken. Die auftretenden elektrischen Felder können Energie speichern und somit einen Kondensator bilden. Je nach Leitungslänge, Pulsform und Frequenz etc. kann das elektrische Feld beziehungsweise dieser Kondensator unterschiedlich groß sein. Bei Signalwechseln kann es durch das notwendige Umladen der Kondensatoren zu einem erhöhten Leistungsbedarf kommen. Bei einer Vielzahl von Leitungen zwischen den einzelnen Detektorpixeln summiert sich der erhöhte Leistungsbedarf der betroffenen elektronischen Schaltungen des CT-Systems und/oder einer Beeinflussung von sensiblen Analogschaltungen im Umfeld führen durch Übersprechen zwischen Leitungen.

Es ist eine Aufgabe der vorliegenden Erfindung, die negativen Einflüsse der Koinzidenzleitungen auf das CT-System zu reduzieren.

Diese Aufgabe wird durch den jeweiligen Gegenstand der unabhängigen Ansprüche gelöst. Vorteilhafte Weiterbildungen und bevorzugte Ausführungsformen sind Gegenstand der abhängigen Ansprüche, der folgenden Beschreibung sowie der Figuren.

Die Erfindung beruht auf der Erkenntnis, dass die Koinzidenzerkennung nicht in jedem Anwendungsfall erforderlich ist, beziehungsweise genutzt wird und/oder je nach Anwendungsfall in unterschiedlicher Ausprägung erforderlich ist beziehungsweise genutzt wird. Daher wird für einen gegebenen Detektorpixel die wahlweise Abkopplung von einem zur Koinzidenzerkennung berücksichtigbaren weiteren Detektorpixel ermöglicht.

Gemäß einem Aspekt der Erfindung wird eine elektronische Schaltung zum Erfassen von Koinzidenzereignissen eines CT-Systems angegeben. Dieses weist einen photonenzählenden Röntgendetektor auf, der ein Detektorpixelarray enthält. Die elektronische Schaltung enthält eine erste Erfassungseinheit, die dazu eingerichtet ist, abhängig von einer mittels eines Detektorpixels des Detektorpixelarrays erfassten Energie ein erstes Auftreffereignissignal zur Verfügung zu stellen. Außerdem enthält die elektronische Schaltung für jedes weitere Detektorpixel wenigstens eines weiteren Detektorpixels des Detektorpixelarrays eine weitere Erfassungseinheit, die dazu eingerichtet ist, abhängig von einer mittels des jeweiligen weiteren Detektorpixels erfassten Energie ein weiteres Auftreffereignissignal zur Verfügung zu stellen. Außerdem enthält die elektronische Schaltung eine Logikschaltung, die dazu eingerichtet ist, das erste Auftreffereignissignal mit dem wenigstens einen weiteren Auftreffereignissignal zu vergleichen und abhängig von einem Ergebnis des Vergleichs ein Koinzidenzsignal zur Verfügung zu stellen. Außerdem enthält die elektronische Schaltung einen Koinzidenzzähler, der dazu eingerichtet ist, abhängig von dem Koinzidenzsignal einen Koinzidenzzählerstand des Koinzidenzzählers zu inkrementieren. Außerdem enthält die elektronische Schaltung für jedes weitere Detektorpixel des wenigstens eines weiteren Detektorpixels eine Schalteinheit, die dazu eingerichtet ist, das jeweilige weitere Auftreffereignissignal von der Logikschaltung zu trennen.

Bei einem photonenzählenden CT-System können die einfallenden Röntgenphotonen auf der aktiven Fläche des Detektorpixels freie Ladungsträger (Elektronen und Löcher) erzeugen, die mit Hilfe eines elektrischen Feldes separiert werden können. Die Ladungen werden beispielsweise in einem starken elektrischen Feld zwischen einer Kathode an der Oberseite und wenigstens einer Anode an der Unterseite des Detektorpixels getrennt. Die Ladung kann beispielsweise mithilfe einer Detektorschaltung des Detektorpixels als analoges Auftreffereignissignal dargestellt werden. Die gemessene Ladung entspricht der elektrischen Energie des Röntgenphotons und kann damit ein Indikator für das Auftreffen eines Röntgenphotons auf der aktiven Fläche des Detektorpixels sein.

Die erste Erfassungseinheit und die wenigstens eine weitere Erfassungseinheit können das jeweilige Auftreffereignissignal in einen digitalen Puls umwandeln, der die Information über ein Auftreffereignis für das jeweilige Detektorpixel kennzeichnen kann. Das jeweilige Auftreffereignissignal wird insbesondere an einem Ausgang der entsprechenden Erfassungseinheit bereitgestellt. Eine gemessene Ladung kann aufgrund unterschiedlicher Effekte auch entstehen, wenn ein Photon nur teilweise oder gar nicht auf die aktive Fläche auftrifft. Eine Weitergabe solcher Ladungen als digitale Pulse kann als falsch-positives Ereignis oder auch als Koinzidenzereignis bezeichnet werden und kann zu Bildfehlern führen.

Zur Erkennung von Koinzidenzereignissen kann die Logikschaltung das erste Auftreffereignissignal des Detektorpixels mit dem wenigstens einen weiteren Auftreffereignissignalen des wenigstens einen weiteren Detektorpixels vergleichen. Wenn das erste Auftreffereignissignal einen Puls aufweist und zum gleichen Zeitpunkt eines des wenigstens einen weiteren Auftreffereignissignals ebenfalls einen Puls aufweist, kann die Logikschaltung dies als ein Koinzidenzereignis detektieren und dies als einen digitalen Puls an ihrem Ausgang kenntlich machen. Mit anderen Worten kann der Ausgang der Logikschaltung beispielsweise auf einer logischen null bleiben, solange kein Koinzidenzsignal erkannt wird. Eine logische eins am Ausgang der Logikschaltung symbolisiert dann beispielsweise ein Koinzidenzereignis.

Der Koinzidenzzähler kann die Koinzidenzereignisse zum Beispiel für einen festgelegten Auslesezeitraum erfassen. Dazu kann der Koinzidenzzählerstand, der am Anfang des Auslesezeitraums initialisiert, das heißt auf null gesetzt wurde, jeweils um eins inkrementiert werden, sobald ein Puls in dem Koinzidenzsignal detektiert wird.

Die wenigstens eine Schalteinheit kann beispielsweise wahlweise zum Verbinden oder Trennen der Verbindung des wenigstens einen weiteren Auftreffereignissignals von der Logikschaltung eingerichtet sein. In anderen Worten kann die jeweilige Schalteinheit das jeweilige weitere Auftreffereignissignal entweder mit der Logikschaltung verbinden, beispielsweise in einem ersten Betriebsmodus des CT-Systems, oder die Verbindung auftrennen, beispielsweise in einem zweiten Betriebsmodus des CT-Systems. Dabei kann insbesondere für den Fall, dass die Anzahl des wenigstens einen weiteren Detektorpixels größer als eins ist, die jeweilige Schalteinheit individuell ein- oder ausgeschaltet werden. Es kann also jedes weitere Auftreffereignissignal des wenigstens einen weiteren Auftreffereignissignals unabhängig voneinander wahlweise mit der Logikschaltung verbunden werden oder von der Logikschaltung getrennt werden.

Im Fall, dass die Anzahl der weiteren Detektorpixel größer eins ist, kann die Logikschaltung beispielsweise die jeweiligen weiteren Auftreffereignissignale zunächst untereinander vergleichen und ein Zwischenergebnis dieses ersten Vergleichs im Anschluss mit dem ersten Auftreffereignissignal in einem zweiten Vergleich vergleichen. Ebenso kann die Logikschaltung beispielsweise zunächst das erste Auftreffereignissignal mit jedem weiteren Auftreffereignissignal des wenigstens einen weiteren Auftreffereignissignals in einem ersten Vergleich vergleichen und im Anschluss einen zweiten Vergleich der jeweiligen Zwischenergebnisse durchführen.

Ein Vergleich von Signalen kann hier und im Folgenden beispielsweise derart verstanden werden, dass die zu vergleichenden Signale einem Logikgatter oder einer Verschaltung von Logikgattern eingangsseitig bereitgestellt werden und ausgangsseitig von dem Logikgatter oder der Verschaltung von Logikgattern ein Ergebnis des Vergleichs erhalten wird.

Beispielsweise kann die wenigstens eine Schalteinheit für jedes weitere Auftreffereignissignal des wenigstens einen weiteren Auftreffereignissignals eine Schalteinheit enthalten, die jeweils dazu eingerichtet ist, das jeweilige weitere Auftreffereignissignal wahlweise von der Logikschaltung zu trennen oder mit dieser zu verbinden. Beispielsweise kann die Logikschaltung für jedes weitere Auftreffereignissignal des wenigstens einen weiteren Auftreffereignissignals einen Eingang aufweisen, der mit dem Ausgang der entsprechenden weiteren Erfassungseinheit verbunden ist, wobei in der jeweiligen Verbindung die jeweilige Schalteinheit angeordnet ist.

Insbesondere kann das Detektorpixel eine aktive Fläche aufweisen, welche die Energie der auf das entsprechende Detektorpixel einfallenden Röntgenphotonen erfassen kann. Dies kann analog ebenso für das wenigstens eine weitere Detektorpixel gelten. Sowohl das Detektorpixel als auch das wenigstens eine weitere Detektorpixel können neben der jeweiligen aktiven Fläche auch weitere elektronische Komponenten aufweisen, insbesondere Komponenten der erfindungsgemäßen elektronischen Schaltung. Die elektronische Schaltung kann auch auf mehrere Detektorpixel verteilt sein, insbesondere auf das Detektorpixel, das wenigstens eine weitere Detektorpixel und/oder auf ein wenigstens nochmals weiteres Detektorpixel. Die elektronische Schaltung kann auch ganz oder teilweise außerhalb des Detektorpixelarrays vorgesehen sein, beispielsweise innerhalb anderer Komponenten des CT-Systems oder als eine externe elektronische Schaltung.

Durch die erfindungsgemäße elektronische Schaltung wird es ermöglicht, die durch die Schaltverbindungen für die Koinzidenzerkennung hervorgerufene parasitäre Kapazität bei Bedarf individuell zu reduzieren. Aufgrund verschiedener Einflussfaktoren kann es innerhalb einer Untersuchung mit einem CT-System vorteilhaft oder erforderlich sein, dass die Koinzidenzerkennung für einzelne Detektorpixel oder für den gesamten Röntgendetektor abgeschaltet werden soll. Es ist auch möglich, dass bei einem Detektorpixel nicht alle sondern nur manche der weiteren Detektorpixel von der Koinzidenzerkennung ausgenommen werden, Durch die erfindungsgemäße elektronische Schaltung kann die Beeinflussung von sensiblen Analogschaltungen im Umfeld reduziert werden und ebenso Blindleistung eingespart werden. Ebenso kann die erfindungsgemäße elektronische Schaltung auch zum gezielten Test der elektronischen Schaltung während der Produktion, z.B. der Produktion einer integrierten Halbleiterschaltung, oder bei Systemtests vorteilhaft sein.

Die beschriebene Verbindung zwischen dem jeweiligen weiteren Auftreffereignissignal und der Logikschaltung kann insbesondere auch reziprok ausgestaltet werden, mit anderen Worten kann das erste Auftreffereignissignal über eine jeweilige weitere Schalteinheit mit jeweils einer weiteren Logikschaltung des wenigstens einen weiteren Detektorpixels verbunden sein. Aus dieser Anordnung ergibt sich jeweils eine schaltbare Sendeleitung und jeweils eine schaltbare Empfangsleitung zwischen dem Detektorpixel und dem wenigstens einem weiteren Detektorpixel.

Gemäß zumindest einer Ausführungsform enthält die erste Erfassungseinheit einen Komparator und/oder enthält die wenigstens eine weitere Erfassungseinheit jeweils einen weiteren Komparator.

Bei einem photonenzählenden CT-System können die einfallenden Röntgenphotonen auf der aktiven Fläche des Detektorpixels freie Ladungsträger (Elektronen und Löcher), die mit Hilfe eines elektrischen Feldes separiert werden. Die Ladungen werden in mindestens einer Ausführungsform in einem starken elektrischen Feld zwischen einer Kathode an der Oberseite und wenigstens einer Anode an der Unterseite des Detektorpixels getrennt. Die Ladung kann beispielsweise mithilfe einer Detektorschaltung des Detektorpixels als analoges Detektorsignal ermittelt werden. Der Komparator ist mit dem analogen Detektorsignal des Detektorpixels verbunden und erhält es eingangsseitig. Der jeweilige weitere Komparator ist mit dem analogen Detektorsignal des jeweiligen weiteren Detektorpixels verbunden und erhält es eingangsseitig.

Ab einer gewissen Ladung kann man beispielsweise von einem Auftreffen oder teilweisen Auftreffen eines Röntgenphotons auf eine aktive Fläche des Detektorpixels ausgehen. Diese Ladung kann man beispielsweise als einen vordefinierten Schwellwert im Komparator beziehungsweise einen vordefinierten weiteren Schwellwert im weiteren Komparator hinterlegen. Der vordefinierte Schwellwert beziehungsweise der vordefinierte weitere Schwellwert kann aber auch einer höheren Energie entsprechen, insbesondere wenn mehrere Komparatoren mit verschiedenen vordefinierten Schwellwerten vorgesehen sind, was auch als spektral aufgelöste Zählung bezeichnet werden kann.

Der Komparator stellt für die elektronische Schaltung die vollständige und zügige Erfassung der relevanten Ereignisse in Bezug auf die Bilddarstellung des Computertomographiesystems dar. Durch den Komparator und den weiteren Komparator ist der Übergang von einem analogen Detektorsignal, das einer Ladung entspricht, in ein digitales Detektorsignal möglich und damit die vorteilhafte Darstellung in einem Histogramm. Die einfache Weiterverarbeitung der Auftreffereignisse von Röntgenphotonen auf die aktive Fläche der Detektorpixel ist ein Vorteil des Einsatzes von Komparatoren.

Gemäß zumindest einer Ausführungsform grenzen das Detektorpixel und jedes weitere Detektorpixel des wenigstens einen weiteren Detektorpixels aneinander.

Mit anderen Worten haben zwei Detektorpixel, die aneinandergrenzen, eine gemeinsame Kante. Diese Nachbarschaft derart aneinandergrenzender Pixel wird in der digitalen Bildverarbeitung als Vierer-Nachbarschaft bezeichnet, da jedes Pixel, das kein Randpixel ist, dann vier Nachbarpixel aufweist. In diesem Fall ist die Anzahl des wenigstens einen weiteren Detektorpixels beispielsweise gleich vier beziehungsweise drei bei einem Randpixel, das kein Eckpixel ist, und zwei bei einem Eckpixel. Die gemeinsamen Kanten erhöhen dabei die Wahrscheinlichkeit eines Koinzidenzereignisses und gelten als besonders relevant, wenn es um die Vermeidung von Fehler aufgrund Koinzidenzereignissen geht.

Gemäß zumindest einer Ausführungsform liegt jedes weitere Detektorpixel des wenigstens einen weiteren Detektorpixels in einer vordefinierten Nachbarschaft des Detektorpixels.

In der digitalen Bildverarbeitung bezeichnet eine Nachbarschaft eine definierte Bildregion um ein Detektorpixel. Mit der Achter-Nachbarschaft existiert eine zweite beispielhafte Nachbarschaft, die in Betracht gezogen werden kann. Bei dieser Nachbarschaft werden zu der Vierer-Nachbarschaft die jeweils diagonal liegenden Detektorpixel betrachtet, die jeweils mit einer Ecke an dem Detektorpixel angrenzen. Darüber hinaus sind auch andere Nachbarschaftsbeziehungen zwischen Detektorpixeln denkbar, die bei bestimmten Anwendungen relevant sein könnten.

Im Speziellen wird auch der Fall des Randproblems betrachtet. Sobald ein Detektorpixel am Rand des Röntgendetektors liegt, ist zum Beispiel eine vollständige Vierer-Nachbarschaft oder eine vollständige Achter-Nachbarschaft gegebenenfalls nicht mehr verfügbar. In diesem Fall werden zum Beispiel nur die Koinzidenzsignale der Detektorpixel betrachtet, die in diesem Randbereich zur Verfügung stehen.

Elektronische Schaltungen zur Erkennung der Koinzidenzereignisse können auf der Kenntnis jedes Detektorpixels von Erkennungen seiner jeweiligen direkten Nachbarpixel basieren. Als direkte Nachbarpixel sollen im Folgenden die Detektorpixel innerhalb einer Vierer-Nachbarschaft gemeint sein. Jedes Detektorpixel hat vier, zwei horizontale und zwei vertikale, direkte Nachbarn. Diese direkten Nachbarpixel zeichnen sich dadurch aus, dass sie mit dem Detektorpixel jeweils eine Pixelkante gemeinsam haben. Sie werden als Vierer-Nachbarn bezeichnet.

Diese Ausführungsformen bietet den Vorteil, dass genau diejenigen Detektorpixel in die Koinzidenzbetrachtung eingeschlossen können oder mit der jeweiligen Schalteinheit individuell getrennt werden können, die für diese Betrachtung die größte Relevanz haben. Je nach ausgewählter Nachbarschaft ergeben sich unterschiedliche Anzahlen an wenigstens einem weiteren Detektorpixel und damit unterschiedliche Anzahlen an Sendeleitungen, Empfangseinheiten und Schalteinheiten.

Gemäß zumindest einer Ausführungsform weist die elektronische Schaltung einen Steuerschaltkreis auf, der dazu eingerichtet ist, einen Schaltzustand der jeweiligen Schalteinheit zu steuern.

Mit anderen Worten kann der Steuerschaltkreis die jeweilige Schalteinheit dazu ansteuern, das jeweilige weitere Auftreffereignissignal entweder mit der Logikschaltung zu verbinden oder die Verbindung aufzutrennen, beispielsweise je nach Betriebsmodus des CT-Systems. Der Steuerschaltkreis kann dafür als eine Zentraleinheit ausgelegt sein, also zum Beispiel als zentrale Steuerung für mehrere Detektorpixel oder für alle Detektorpixel des Röntgendetektors. Möglich ist aber auch ein modular aufgebauter Steuerschaltkreis, der zum Beispiel für die Steuerung für wenige Detektorpixel oder für ein Detektorpixel des Röntgendetektors eingerichtet ist.

Ein Vorteil des Steuerschaltkreises ist der zentrale Zugriff auf die wenigstens eine Schalteinheit und damit auf die Verbindung zwischen dem wenigstens einen weiteren Auftreffereignissignal und der Logikschaltung. Damit ist eine schnelle und abgestimmte Konfiguration des CT-Systems möglich.

Gemäß zumindest einer Ausführungsform enthält die jeweilige Schalteinheit einen Multiplexer, der dazu eingerichtet ist, der Logikschaltung alternativ zu dem jeweiligen weiteren Auftreffereignissignal ein Alternativsignal zur Verfügung zu stellen.

Ein Multiplexer ist eine Selektionsschaltung in der analogen und digitalen Elektronik, mit der insbesondere aus einer Anzahl von Eingangssignalen eines ausgewählt und an den Ausgang des Multiplexers durchgeschaltet werden kann. Beispielsweise kann der jeweilige Multiplexer zwischen dem jeweiligen weiteren Auftreffereignissignal und dem jeweiligen Alternativsignal umschalten. Ein erster Eingang des Multiplexers ist insbesondere mit dem jeweiligen weiteren Auftreffereignissignal beschaltet und ein zweiter Eingang des Multiplexers ist insbesondere mit dem jeweiligen Alternativsignal beschaltet. Sofern vorhanden kann wenigstens ein weiterer Eingang des Multiplexers auch unbeschaltet sein, das heißt ohne ein definiertes Bezugspotential.

Wie bereits oben beschrieben kann auch diese Ausführungsform reziprok aufgebaut sein, das heißt, dass die weitere Schalteinheit in manchen Ausführungsformen ebenso einen Multiplexer enthält, der dazu eingerichtet ist, alternativ zu dem ersten Auftreffereignissignal wenigstens ein erstes Alternativsignal zur Verfügung zu stellen.

Ein Vorteil dieser Ausführungsformen besteht darin, dass die Möglichkeiten zur Signalführung flexibel gestaltet werden können. Der jeweilige Multiplexer kann je nach Konfiguration dazu eingerichtet werden, die jeweils notwendigen Signale an die Logikschaltung weiterzugeben.

Gemäß zumindest einer Ausführungsform ist die jeweilige Schalteinheit dazu eingerichtet, der Logikschaltung alternativ zu dem jeweiligen weiteren Auftreffereignissignal jeweils ein Alternativsignal oder ein konstantes Bezugspotential zur Verfügung zu stellen.

Mit anderen Worten kann die jeweilige Schalteinheit der Logikschaltung alternativ zu dem jeweiligen weiteren Auftreffereignissignal zum Beispiel ein Massepotential oder ein vordefiniertes anderes Spannungspotential zur Verfügung stellen. Dazu kann die jeweilige Schalteinheit das jeweilige weitere Auftreffereignissignal von der Logikschaltung trennen. Diese Alternative schaltet für die entsprechende Verbindung die Koinzidenzerkennung ab. Außerdem kann die wenigstens eine Schalteinheit das konstante Bezugspotential mit der Logikschaltung verbinden.

Im Falle des Einsatzes eines Multiplexers wie beschrieben kann das wenigstens eine Alternativsignal dem konstanten Bezugspotential entsprechen.

Ein Vorteil dieser Ausführungsformen besteht darin, dass die entsprechende Leitung im Schaltzustand mit dem konstanten Bezugspotential beispielsweise keine Pulse aufweist und damit die leitungsgebundene parasitäre Kapazität der Leitung reduziert werden kann.

Gemäß zumindest einer Ausführungsform enthält die elektronische Schaltung eine zweite Erfassungseinheit, die dazu eingerichtet ist, abhängig von der mittels des Detektorpixels erfassten Energie ein zweites Auftreffereignissignal zur Verfügung zu stellen, und die elektronische Schaltung enthält einen Auftreffereigniszähler, der dazu eingerichtet ist, abhängig von dem zweiten Auftreffereignissignal einen Zählerstand des Auftreffereigniszählers zu inkrementieren.

Mit anderen Worten wird für das Detektorpixel eine zweite Erfassungseinheit angegeben, die die mittels des Detektorpixels erfasste Energie auswerten kann. Die zweite Erfassungseinheit kann im Besonderen einen zweiten Komparator enthalten, der mit einem zweiten vordefinierten Schwellwert konfiguriert ist. Dieser zweite vordefinierte Schwellwert kann gleich oder auch unterschiedlich zum vordefinierten Schwellwert sein. Somit lässt sich eine Redundanz beziehungsweise eine weitere spektrale Auflösung erreichen.

Der Auftreffereigniszähler kann die gleiche Funktionsweise wie der Koinzidenzzähler aufweisen. Der Auftreffereigniszähler kann die Auftreffereignisse zum Beispiel in dem Auslesezeitraum erfassen. Dazu kann ein weiterer Zählerstand des Auftreffereigniszählers, der am Anfang des Auslesezeitraums initialisiert, das heißt auf null gesetzt wurde, jeweils um eins inkrementiert werden, sobald ein Puls in dem zweiten Auftreffereignissignal detektiert wird.

Ein Vorteil dieser Ausführungsformen liegt darin, dass bei aktivierter Koinzidenzerkennung, also bei hergestellter Verbindung des jeweiligen weiteren Auftreffereignissignals zur Logikeinheit durch die jeweilige Schalteinheit, gleichzeitig auch eine Bestimmung der absoluten Auftreffereignisse für unterschiedliche Schwellwerte stattfinden kann. Aus der Information über die absoluten Auftreffereignisse zusammen mit der Information über die Koinzidenzereignisse im gleichen Auslesezeitraum können Bilddaten verbessert werden.

Gemäß zumindest einer weiteren Ausführungsform ist die elektronische Schaltung als ASIC (englisch: application-specific integrated circuit) oder als sonstige integrierte Schaltung ausgeführt.

In der digitalen Schaltungstechnik sind verschiedene Arten von integrierte Schaltungen bekannt, die für diese Anwendung denkbar sind, insbesondere kann ein ASIC verwendet werden, aber auch andere Arten.

Auch sind andere Formen von integrierten Schaltungen für diese Ausführung denkbar.

Der ASIC oder die integrierte Schaltung kann dabei alle vorgestellten Komponenten der elektronischen Schaltung, also insbesondere die aktive Fläche, den Komparator, den weiteren Komparator, den Zähler, den weiteren Zähler, das Register, das weitere Register und den wenigstens einen Schaltungsteil enthalten, oder nur eine Untermenge in modulartigem Aufbau enthalten, soweit diese Komponenten in den entsprechenden Ausführungsformen vorgesehen sind.

Diese Ausführungsform bietet den Vorteil einer kompakten Bauweise bei entsprechend hoher Leistungsfähigkeit. Die hohen Anforderungen an die Anzahl der Detektorpixel und die Geschwindigkeit der Datenverarbeitung sind so besser zu erfüllen.

Gemäß zumindest einer Ausführungsform enthält die elektronische Schaltung eine Umschalteinheit, welche dazu eingerichtet ist, dem Koinzidenzzähler wahlweise entweder das erste Auftreffereignissignal oder das Koinzidenzsignal zur Verfügung stellen.

Mit anderen Worten ist es durch die Umschalteinheit möglich, mit dem Koinzidenzzähler entweder Koinzidenzereignisse zu zählen oder alternativ die Auftreffereignisse mittels des ersten Auftreffereignissignals. Die Auftreffereignisse aus dem ersten Auftreffereignissignal können dann im Koinzidenzzähler zusätzlich zu den Auftreffereignissen aus dem zweiten Auftreffereignissignal im Auftreffereigniszähler für den gleichen Auslesezeitraum zur Verfügung stehen.

Ein Vorteil dieser Ausführungsformen ist, dass, wenn die Umschalteinheit das erste Auftreffereignissignal am Koinzidenzzähler zur Verfügung stellt, zum Beispiel die Anzahl der Auftreffereignisse von Röntgenphotonen mit unterschiedlichen Energieschwellen bei abweichenden Schwellwerten gleichzeitig erfasst werden können. Alternativ kann bei gleichen Schwellwerten die Redundanz des Systems erhöht werden.

Gemäß zumindest einer Ausführungsform enthält die Logikschaltung ein ODER-Gatter, das eingangsseitig mit dem wenigstens einen weiteren Auftreffereignissignal verbunden ist und das dazu eingerichtet ist, an einem Ausgang des ODER-Gatters ein Nachbarereignissignal zur Verfügung zu stellen, und ein UND-Gatter, das eingangsseitig mit dem ersten Auftreffereignissignal und dem Nachbarereignissignal verbunden ist und das dazu eingerichtet ist, an einem Ausgang des UND-Gatters das Koinzidenzsignal zur Verfügung zu stellen.

Mit anderen Worten kann durch die beschriebene Anordnung des ODER-Gatters und des UND-Gatters eine Prüfung auf ein gleichzeitiges Auftreten von Pulsen auf dem ersten

Auftreffereignissignal und dem wenigstens einen weiteren Auftreffereignissignal realisiert werden. Das ODER-Gatter kann für jedes des wenigstens einen weiteren Auftreffereignissignals jeweils einen Eingang enthalten und kann die digitalen Signale auswerten. Das Ergebnis kann am Ausgang des ODER-Gatters als Nachbarereignissignal zur Verfügung stellt werden. Eine logische eins auf dem Nachbarereignissignal kann also bedeuten, dass eines der wenigstens einen weiteren Detektorpixel ein Auftreffereignis detektiert hat. Am UND-Gatter kann die Information des wenigstens einen weiteren Auftreffereignissignals mit der des ersten Auftreffereignissignals logisch verknüpft werden.

Die Verwendung von ODER-Gattern und von UND-Gattern ist besonders vorteilhaft, da die entsprechenden Schaltungen schnell und zuverlässig die notwendigen Ergebnisse erzielen.

Die Begriffe UND-Gatter und ODER-Gatter können insbesondere funktional verstanden werden, d.h. es muss nicht unbedingt jeweils ein einzelnes Gatter sein, sondern es kann auch eine Auslegung mit anderen Gatterarten sein, insbesondere mit NOR-Gattern bzw. NAND-Gattern. Mit anderen Worten kann ein UND-Gatter auch als UND-Schaltung bezeichnet werden und ein ODER-Gatter kann als ODER-Schaltung bezeichnet werden.

Es ist jedoch auch möglich andere logische Schaltungen zu nutzen, welche dieselbe Funktion, insbesondere Wahrheitstabelle, aufweisen wie die Kombination aus dem ODER-Gatter und dem UND-Gatter.

Gemäß einem weiteren Aspekt der Erfindung wird ein photonenzählender Röntgendetektor für ein CT-System angegeben, welcher eine erfindungsgemäße elektronische Schaltung enthält.

Weitere Ausführungsformen des erfindungsgemäßen Röntgendetektors folgen unmittelbar aus den verschiedenen Ausgestaltungen der erfindungsgemäßen elektronischen Schaltung. Insbesondere lassen sich einzelne Merkmale und entsprechende Erläuterungen sowie Vorteile bezüglich der verschiedenen Ausführungsformen zu der erfindungsgemäßen Verfahren analog auf entsprechende Ausführungsformen des erfindungsgemäßen Röntgendetektors übertragen.

Gemäß einem weiteren Aspekt der Erfindung wird ein CT-System angegeben, welches eine Röntgenröhre zum Aussenden von Röntgenphotonen, einen erfindungsgemäßen photonenzählenden Röntgendetektor und ein Datenverarbeitungssystem enthält, das dazu eingerichtet ist, CT-Bilddaten abhängig von dem Koinzidenzzählerstand zu erzeugen.

Weitere Ausführungsformen des erfindungsgemäßen CT-Systems folgen unmittelbar aus den verschiedenen Ausgestaltungen der erfindungsgemäßen elektronischen Schaltung.

Insbesondere lassen sich einzelne Merkmale und entsprechende Erläuterungen sowie Vorteile bezüglich der verschiedenen Ausführungsformen zu der erfindungsgemäßen Verfahren analog auf entsprechende Ausführungsformen des erfindungsgemäßen CT-Systems übertragen.

Gemäß einem weiteren Aspekt der Erfindung wird ein Verfahren zum Erfassen von Koinzidenzereignissen eines CT-Systems, welches einen photonenzählenden Röntgendetektor aufweist, der ein Detektorpixelarray enthält, angegeben. Dabei wird abhängig von einer mittels eines Detektorpixels des Detektorpixelarrays erfassten Energie ein erstes Auftreffereignissignal zur Verfügung gestellt. Außerdem wird für jedes weitere Detektorpixel wenigstens eines weiteren Detektorpixels des Detektorpixelarrays abhängig von einer mittels des jeweiligen weiteren Detektorpixels erfassten Energie ein weiteres Auftreffereignissignal zur Verfügung gestellt. Außerdem wird das erste Auftreffereignissignal mittels einer Logikschaltung, insbesondere einer Logikschaltung des Röntgendetektors, mit dem wenigstens einen weiteren Auftreffereignissignal verglichen und abhängig von einem Ergebnis des Vergleichs ein Koinzidenzsignal zur Verfügung gestellt. Abhängig von dem Koinzidenzsignal wird ein Koinzidenzzählerstand inkrementiert. In einem ersten Betriebsmodus des CT-Systems wird jedes weitere Auftreffereignissignal des wenigstens einen weiteren Auftreffereignissignals der Logikschaltung eingangsseitig zur Verfügung gestellt. In einem zweiten Betriebsmodus des CT-Systems wird zumindest ein weiteres Auftreffereignissignal des wenigstens einen weiteren Auftreffereignissignals von der Logikschaltung getrennt.

Gemäß zumindest einer Ausführungsform des Verfahrens wird am Ende eines vorgegebenen Auslesezeitraums ein finaler Koinzidenzzählerstand des Koinzidenzzählers bestimmt und es werden abhängig von dem finalen Koinzidenzzählerstand CT-Bilddaten erzeugt.

Gemäß zumindest einer Ausführungsform des Verfahrens wird abhängig von der mittels des Detektorpixels erfassten Energie eine Anzahl von Auftreffereignissen während des Auslesezeitraums bestimmt und es werden die CT-Bilddaten abhängig von dem finalen Koinzidenzzählerstand und der Anzahl von Auftreffereignissen während des Auslesezeitraums erzeugt.

Weitere Ausführungsformen des erfindungsgemäßen Verfahrens folgen unmittelbar aus den verschiedenen Ausgestaltungen der erfindungsgemäßen elektronischen Schaltung und umgekehrt. Insbesondere lassen sich einzelne Merkmale und entsprechende Erläuterungen sowie Vorteile bezüglich der verschiedenen Ausführungsformen zu der erfindungsgemäßen elektronischen Schaltung analog auf entsprechende Ausführungsformen des erfindungsgemäßen Verfahrens übertragen. Insbesondere ist die erfindungsgemäße elektronische Schaltung zum Durchführen eines erfindungsgemäßen Verfahrens ausgebildet oder programmiert. Insbesondere führt die erfindungsgemäße elektronische Schaltung das erfindungsgemäße Verfahren durch.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

Dabei zeigen
- FIG 1: ein schematisches Blockschaltdiagramm einer beispielhaften Ausführungsform einer erfindungsgemäßen elektronischen Schaltung zum Erfassen von Koinzidenzereignissen eines Computertomographiesystems;
- FIG 2: ein schematisches Blockschaltdiagramm einer weiteren beispielhaften Ausführungsform einer erfindungsgemäßen elektronischen Schaltung;
- FIG 3: ein schematisches Blockschaltdiagramm einer weiteren beispielhaften Ausführungsform einer erfindungsgemäßen elektronischen Schaltung;
- FIG 4: ein schematisches Blockschaltdiagramm einer weiteren beispielhaften Ausführungsform einer erfindungsgemäßen elektronischen Schaltung; und
- FIG 5: ein schematisches Blockschaltdiagramm einer Logikschaltung einer weiteren beispielhaften Ausführungsform einer erfindungsgemäßen elektronischen Schaltung.

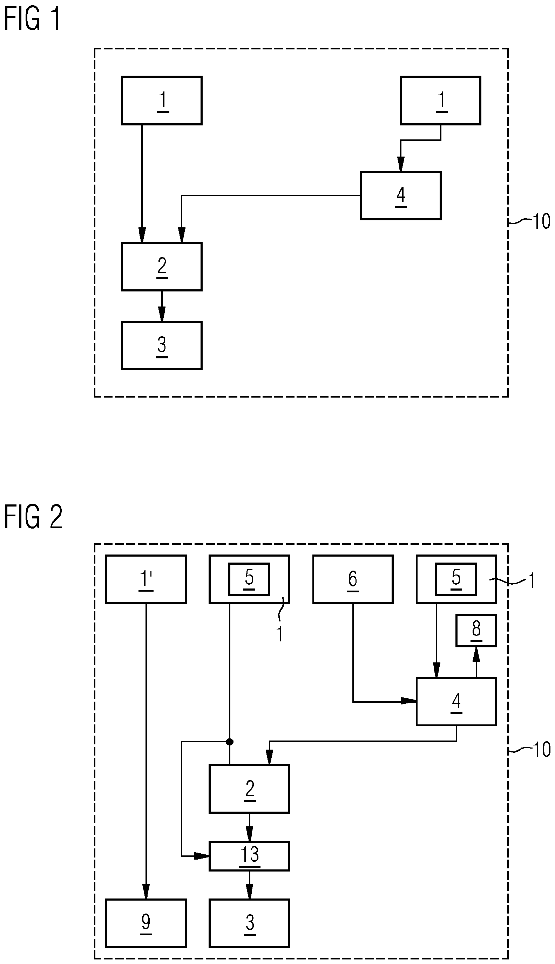

In FIG 1 ist ein Blockschaltdiagramm einer erfindungsgemäßen elektronischen Schaltung 10 zum Erfassen von Koinzidenzereignissen eines Computertomographiesystems, CT-Systems, welches einen photonenzählenden Röntgendetektor aufweist, der ein Detektorpixelarray enthält, gezeigt.

Die elektronische Schaltung 10 enthält eine erste Erfassungseinheit 1 für ein Detektorpixel 11 und eine weitere Erfassungseinheit 1 für ein weiteres Detektorpixel 12 des wenigstens einen weiteren Detektorpixels 12. Die Anzahl des wenigstens einen weiteren Detektorpixels 12 ist in dieser Figur zur einfacheren Darstellung beispielhaft gleich eins. Dies dient lediglich der grundlegenden Funktionsweise und stellt keine Limitierung der Erfindung dar. Insbesondere kann die Anzahl des wenigstens einen weiteren Detektorpixels 12 gleich eins oder größer als eins sein.

Die erste Erfassungseinheit 1 ist dazu eingerichtet, abhängig von einer mittels des Detektorpixels 11 erfassten Energie ein erstes Auftreffereignissignal zur Verfügung zu stellen. Dazu ist die erste Erfassungseinheit 1 insbesondere mit der aktiven Fläche des Detektorpixels 11, die die Energie der einfallenden Röntgenphotonen des Detektorpixels 11 erfassen kann, verbunden. Diese aktive Fläche ist zur einfacheren Darstellung auf FIG 1 und auf den folgenden Figuren nicht enthalten. Gleiches gilt für die weitere Erfassungseinheit 1 und die zweite Erfassungseinheit 1'.

Die erste Erfassungseinheit 1 ist ausgangsseitig mit einer Logikschaltung 2 verbunden. Die weitere Erfassungseinheit 1 ist ausgangsseitig mit einer Schalteinheit 4 verbunden. Die Schalteinheit 4 ist ausgangsseitig mit der Logikschaltung 2 verbunden. Mit anderen Worten ist die weitere Erfassungseinheit 1 ausgangsseitig über die Schalteinheit 4 mit der Logikschaltung 2 verbunden. Die Logikschaltung 2 erhält also das erste Auftreffereignissignal direkt und das weiteres Auftreffereignissignal über die Schalteinheit 4.

Die Schalteinheit 4 ist dazu eingerichtet, die Verbindung zwischen der weiteren Erfassungseinheit 1 und der Logikschaltung 2 wahlweise zu trennen oder herzustellen, sodass wahlweise das weitere Auftreffereignissignal an die Logikschaltung 2 übertragen wird oder nicht übertragen wird. Die Logikschaltung 2 kann eine Vergleichslogik der beiden Auftreffereignissignale, insbesondere dem ersten Auftreffereignissignal und dem weiteren Auftreffereignissignal, durchführen. Zum Beispiel kann die Schalteinheit 4 bei getrennter Verbindung ein Signal in Form einer logischen null, ein Signal in Form einer logischen eins oder ein anderes Signal in Form eines offenen Zustands, beispielsweise hochohmig oder niederohmig, an die Logikschaltung 2 weitergeben. Dadurch ist ein nicht stromverbrauchender Zustand realisiert, der je nach verwendeter Logikfamilie unterschiedlich ausgestaltet sein kann. Das Ergebnis der Vergleichslogik kann als ein Koinzidenzsignal von der Logikschaltung 2 an einen Koinzidenzzähler 3 weitergegeben werden.

Die Logikschaltung 2 kann beispielsweise für den dargestellten Fall, in dem die Anzahl des wenigstens einen weiteren Detektorpixels 12 gleich 1 ist, ein UND-Gatter 16 enthalten oder aus einem UND-Gatter bestehen. Daraus kann sich bei gleichzeitigem Auftreten eines Pulses auf dem ersten Auftreffereignissignal und auf dem weiteren Auftreffereignissignal und eingeschalteter Schalteinheit 4 aufgrund des UND-Gatters 16 in der Logikschaltung 2 ebenfalls ein Puls auf dem Koinzidenzsignal ergeben.

Der Koinzidenzzähler 3 kann ein auftretendes Koinzidenzereignis in Form eines Pulses im Koinzidenzsignal erfassen und einen Koinzidenzzählerstand bei jeder Erfassung eines weiteren Pulses um eins erhöhen. Sobald die Schalteinheit 4 die Verbindung des weiteren Auftreffereignissignals von der Logikschaltung 2 trennt, kann das Koinzidenzsignal im dargestellten Beispiel, in dem die Anzahl des wenigstens einen weiteren Detektorpixels 12 gleich eins ist, gleich null sein.

In einem Ausführungsbeispiel kann die Funktion der Schalteinheit 4 als Aktivierung bzw. Deaktivierung einer Koinzidenzerkennung verstanden werden. Im eingeschalteten Zustand der Schalteinheit 4 kann der Koinzidenzzähler 3 die Anzahl der Koinzidenzereignisse zwischen dem Detektorpixel 11 und dem weiteren Detektorpixel 12 erfassen. Im ausgeschalteten Zustand der Schalteinheit 4 ist es möglich, dass der Koinzidenzzähler 3 keine Auftreffereignisse erfasst.

Die erste Erfassungseinheit 1 kann beispielsweise einen Komparator 5 enthalten. Der Komparator 5 kann beispielsweise das analoge Detektorsignal des Detektorpixels 11 eingangsseitig erhalten und den Wert mit einem vordefinierten Schwellwert vergleichen. Der Komparator 5 kann einen digitalen Puls ausgeben, wenn der vordefinierte Schwellwert erreicht oder überschritten wird. Dieser Puls wird dann als erstes Auftreffereignissignal an die Logikschaltung 2 weitergegeben.

In FIG 2 ist ein weiteres Ausführungsbeispiel der erfindungsgemäßen elektronischen Schaltung 10 gezeigt, das auf dem Ausführungsbeispiel der FIG 1 basiert. Darin sind die erste Erfassungseinheit 1, die weitere Erfassungseinheit 1 und eine zweite Erfassungseinheit 1' dargestellt. Das in FIG 2 gezeigte Ausführungsbeispiel kann alle Merkmale aus FIG 1 aufweisen. Insbesondere wird auch in FIG 2 ein Beispiel gezeigt, bei dem die Anzahl des mindestens einen weiteren Detektorpixels 12 gleich eins ist.

Die erste Erfassungseinheit 1 ist mit der Logikschaltung 2 und beispielsweise mit einer Umschalteinheit 13 verbunden. Wie oben ist die weitere Erfassungseinheit 1 über die Schalteinheit 4 mit der Logikschaltung 2 verbunden.

Ein Steuerschaltkreis 6 kann die Schalteinheit 4 steuern, also beispielsweise einen Schaltzustand der Schalteinheit 4 verändern. Beispielsweise kann ein Alternativsignal 8, zum Beispiel ein konstantes Massepotential, ebenfalls mit der Schalteinheit 4 verbunden sein. Der Steuerschaltkreis 6 kann beispielsweise die Schalteinheit 4 entsprechend ansteuern, sodass die Schalteinheit 4 wahlweise das weitere Auftreffereignissignal oder das Alternativsignal 8 am Ausgang der Schalteinheit 4 anliegt. Dieser Ausgang ist mit der Logikschaltung 2 verbunden. Die Schalteinheit 4 kann insbesondere auch einen Multiplexer 7 enthalten. Der Multiplexer 7 kann wahlweise eines der eingangsseitig angeschlossenen Signale, also das weitere Auftreffereignissignal oder das Alternativsignal 8, an die Logikschaltung 2 weitergeben.

Der Ausgang der Logikschaltung 2 ist mit einem Eingang der Umschalteinheit 13 verbunden und der Ausgang der Umschalteinheit 13 mit dem Koinzidenzzähler 3. Die Umschalteinheit 13 kann in einem Ausführungsbeispiel wahlweise das Koinzidenzsignal der Logikschaltung 2 oder das erste Auftreffereignissignal an ihrem Ausgang ausgeben. Dieser Ausgang ist mit dem Koinzidenzzähler 3 verbunden.

Die weitere Erfassungseinheit 1 kann einen weiteren Komparator 5 enthalten. Dieser weitere Komparator 5 kann sich analog zu dem Komparator 5 verhalten. Der weitere Komparator 5 kann beispielsweise das analoge Detektorsignal des jeweils einen weiteren Detektorpixels 12 erhalten und mit einem vordefinierten weiteren Schwellwert vergleichen. Der weitere Komparator 5 kann einen digitalen Puls ausgeben, wenn der vordefinierte weitere Schwellwert erreicht oder überschritten wird. Dieser Puls wird dann als weiteres Auftreffereignissignal an die Schalteinheit 4 weitergegeben.

Die zweite Erfassungseinheit 1' ist mit einem Auftreffereigniszähler 9 verbunden. Der Auftreffereigniszähler 9 kann beispielsweise die Auftreffereignisse des Detektorpixels 11 zählen. Die zweite Erfassungseinheit 1' kann ebenso einen zweiten Komparator 5 enthalten. Der zweite Komparator 5 kann das analoge Detektorsignal des Detektorpixel 11 mit einem vordefinierten zweiten Schwellwert vergleichen und kann einen Puls ausgeben, wenn der vordefinierte zweite Schwellwert erreicht oder überschritten wird. Insbesondere kann der vordefinierte erste Schwellwert von dem vordefinierten zweiten Schwellwert abweichen und damit können die beiden Komparatoren, der erste und der zweite Komparator, unterschiedliche Signale ausgeben, auch wenn sie eingangsseitig das gleiche Detektorsignal erhalten.

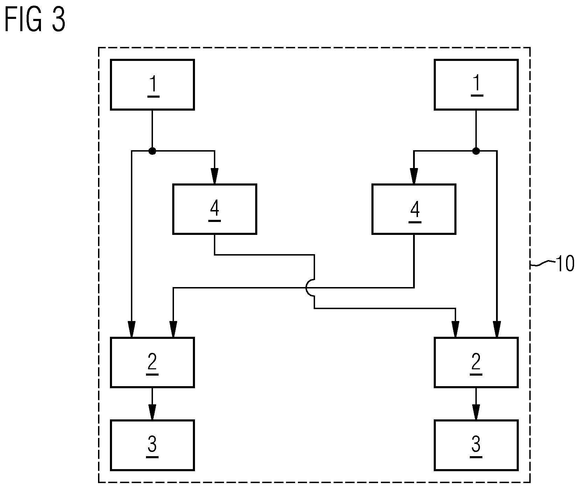

FIG 3 zeigt ein Blockschaltdiagramm eines Ausführungsbeispiels, bei dem die bezüglich FIG 1 beschriebene elektronische Schaltung 10 reziprok aufgebaut ist. Dabei ist beispielsweise die erste Erfassungseinheit 1 über eine weitere Schalteinheit 4 mit einer weiteren Logikschaltung 2 verbunden. Das Detektorpixel 11 kann also an der Logikschaltung 2 jeweils das weitere Auftreffereignissignal von jeweils einem weiteren Detektorpixel 12 erhalten, wie umgekehrt, das jeweils eine weitere Detektorpixel 12 das erste Auftreffereignissignal des Detektorpixels 11 an der jeweiligen weiteren Logikschaltung 2 erhalten kann.

Auch in diesem Blockschaltdiagramm ist zur einfacheren Darstellung die Anzahl des wenigstens einen weiteren Detektorpixels 12 mit eins gewählt.

Aus der Darstellung in FIG 3 kann eine beispielhafte Beziehung zwischen zwei Detektorpixeln 11, 12 zur Koinzidenzerkennung entnommen werden. Zwei Detektorpixel 11, 12 können in einem Ausführungsbeispiel jeweils eine abschaltbare Datenleitung in jede Richtung aufweisen, insbesondere können sie jeweils eine abschaltbare Datenleitung zum jeweils anderen Detektorpixel 11, 12 hin aufweisen und eine abschaltbare Datenleitung vom jeweils anderen Detektorpixel 11, 12 weg aufweisen.

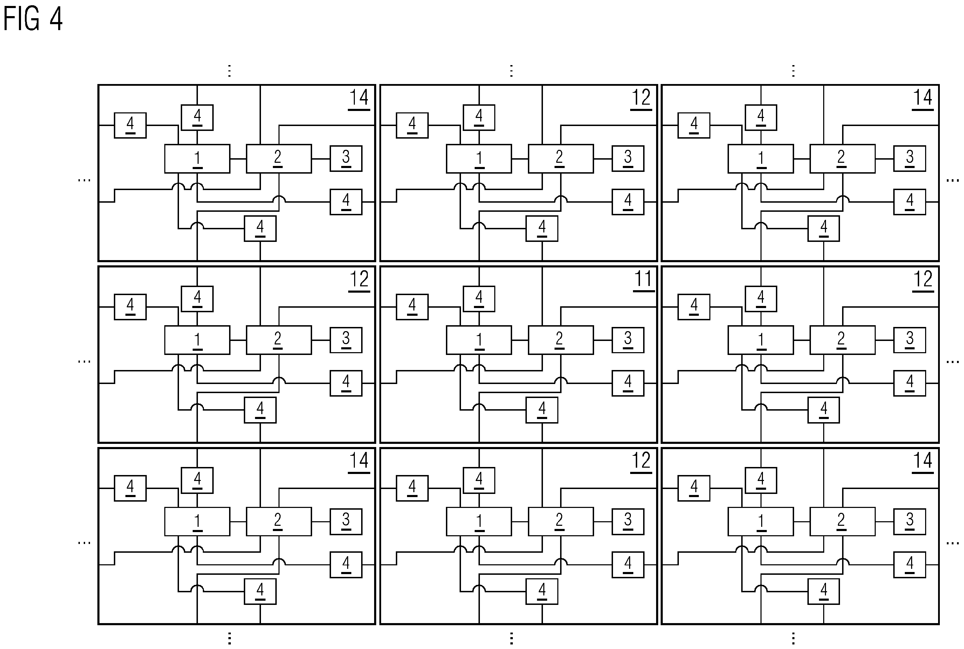

FIG 4 zeigt ein Blockschaltdiagramm eines weiteren Ausführungsbeispiels einer erfindungsgemäßen elektronischen Schaltung 10. Dabei kann die dargestellte elektronische Schaltung 10 die gleichen Merkmale der in den anderen Figuren dargestellten elektronischen Schaltung 10 aufweisen. Insbesondere kann die elektronische Schaltung 10 in dieser Darstellung auf mehrere Detektorpixel 11, 12 verteilt sein.

Die FIG 4 zeigt mittig eine erste Anzahl von Komponenten der elektronischen Schaltung 10, die dem Detektorpixel 11 zugeordnet werden können. In dieser Darstellung ist die Anzahl des wenigstens einen weiteren Detektorpixels 12 beispielhaft mit vier angegeben. Es ergibt sich damit beispielsweise die oben beschriebene Vierer-Nachbarschaft. Außerdem ist eine zweite Anzahl von Komponenten der elektronischen Schaltung 10 dargestellt, die jeweils den vier weiteren Detektorpixel 12 zugeordnet werden können.

Die erste Anzahl der Komponenten enthält in einem Ausführungsbeispiel die erste Erfassungseinheit 1, die Logikschaltung 2, den Koinzidenzzähler 3 und vier weitere Schalteinheiten 4, die jeweils das erste Auftreffereignissignal an die benachbarten weiteren Detektorpixel 12 verbinden. Die jeweiligen weiteren Auftreffereignissignale der vier weiteren Detektorpixel 12 können über die jeweiligen Schalteinheiten 4 mit der Logikschaltung 2 des Detektorpixels 11 verbunden sein.

In FIG 4 sind außerdem beispielhaft vier diagonale Detektorpixel 14 dargestellt. Diese diagonalen Detektorpixel haben in einem Ausführungsbeispiel keine direkte Verbindung zum Zwecke der Koinzidenzerkennung zu dem Detektorpixel 11. In einem anderen Ausführungsbeispiel ist auch denkbar, die vorgestellte Systematik auf die Achter-Nachbarschaft auszuweiten, wobei dann die diagonalen Detektorpixel 14 und die weiteren Detektorpixel 12 mit dem Detektorpixel 11 verbunden sein können.

Die in jede Richtung dargestellten drei Punkte zeigen die Fortsetzung dieser Anordnung. Die Verbindungen können beispielhaft über das Detektorpixelarray hinweg analog aufgebaut werden. Dabei ist zu beachten, dass diejenigen Detektorpixel 11, die am Rand des Detektorpixelarrays liegen, eine reduzierte Anzahl an Nachbarn haben können. Sollte das Detektorpixel 11 am Rand und nicht an einer Ecke liegen, reduziert sich die Anzahl der weiteren Detektorpixel 12 auf drei, die Anzahl der diagonalen Detektorpixel 14 auf zwei, die Achter-Nachbarschaft auf fünf. Sollte das Detektorpixel 11 in einer Ecke liegen, reduziert sich die Anzahl der weiteren Detektorpixel 12 auf zwei, die Anzahl der diagonalen Detektorpixel 14 auf eins, die Achter-Nachbarschaft auf drei.

Aus der Darstellung wird auch die Anzahl der Auftreffereignissignale deutlich, die beispielhaft zum Erfassen von Koinzidenzereignissen bei einem CT-System mit einer typischen Anzahl an Detektorpixeln von größer 1 Million notwendig sind. Diese können wie in der Erfindung beschrieben individuell anhand der Vielzahl der Schalteinheit 4 und der Vielzahl der weiteren Schalteinheit 4 von dem wenigstens einem weiteren Detektorpixel getrennt werden.

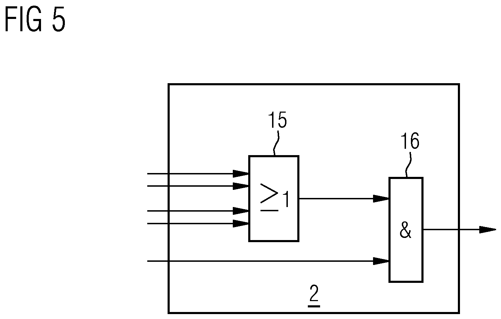

FIG 5 zeigt die Logikschaltung 2 eines weiteren Ausführungsbeispiels der erfindungsgemäßen elektronischen Schaltung 10. Die Logikschaltung 2 kann beispielsweise ein ODER-Gatter 15 und ein UND-Gatter 16 enthalten.

Das ODER-Gatter 15 kann das wenigstens eine weitere Auftreffereignissignal eingangsseitig erhalten. Das dargestellte Beispiel zeigt eine Anzahl von vier für das wenigstens eine weitere Detektorpixel 12 und dementsprechend vier weitere Auftreffereignissignale an den Eingängen des ODER-Gatters 15. Ebenso kann in einem anderen Ausführungsbeispiel die Anzahl des wenigstens einen weiteren Detektorpixels 12 größer sein, zum Beispiel acht für den Fall, dass die diagonalen Detektorpixel 14 in die Koinzidenzerkennung einbezogen werden, oder auch kleiner sein. Dementsprechend würde das ODER-Gatter 15 dann mehr als vier weitere Auftreffereignissignale eingangsseitig erhalten.

Das ODER-Gatter 15 kann an seinem Ausgang ein Nachbarereignissignal zur Verfügung stellen. Im dargestellten Beispiel ist das Nachbarereignissignal eingangsseitig mit dem UND-Gatter verbunden. Für den Fall, dass die Anzahl des wenigsten einen weiteren Detektorpixels gleich eins ist, kann auf das ODER-Gatter 15 verzichtet werden, dann ist insbesondere das weitere Auftreffereignissignal gleich dem Nachbarereignissignal.

Das UND-Gatter 16 erhält eingangsseitig das erste Auftreffereignissignal und das Nachbarereignissignal. Am Ausgang kann das UND-Gatter 16 somit das Koinzidenzsignal zur Verfügung stellen.

Insbesondere kann die Anordnung des ODER-Gatters 15 und des UND-Gatters 16 auch gewissermaßen invertiert werden, also die Reihenfolge der Verbindungen getauscht werden. Dabei kann beispielsweise die Anzahl der UND-Gatter 16 der Anzahl des wenigstens einen weiteren Auftreffereignissignals entsprechen. Jedes UND-Gatter 16 kann dann mit jeweils einem des wenigstens einen weiteren Auftreffereignissignals eingangsseitig verbunden sein. Außerdem kann jedes UND-Gatter 16 das erste Auftreffereignissignal eingangsseitig zur Verfügung gestellt bekommen. Die Ausgänge der UND-Gatter 16 können dann eingangsseitig mit dem ODER-Gatter 15 verbunden sein. Der Ausgang des ODER-Gatters 15 entspricht in diesem Ausführungsbeispiel dem Koinzidenzsignal. Es sind auch andere Ausgestaltungen der Logikschaltung 2 möglich.

Unabhängig vom grammatikalischen Geschlecht eines bestimmten Begriffes sind Personen mit männlicher, weiblicher oder anderer Geschlechteridentität mit umfasst.

## Patentansprüche

1. Elektronische Schaltung (10) zum Erfassen von Koinzidenzereignissen eines Computertomographiesystems, CT-Systems, welches einen photonenzählenden Röntgendetektor aufweist, der ein Detektorpixelarray enthält, wobei die elektronische Schaltung (10)
- eine erste Erfassungseinheit (1) enthält, die dazu eingerichtet ist, abhängig von einer mittels eines Detektorpixels (11) des Detektorpixelarrays erfassten Energie ein erstes Auftreffereignissignal zur Verfügung zu stellen,
- für jedes weitere Detektorpixel (12) wenigstens eines weiteren Detektorpixels (12) des Detektorpixelarrays eine weitere Erfassungseinheit (1) enthält, die dazu eingerichtet ist, abhängig von einer mittels des jeweiligen weiteren Detektorpixels (12) erfassten Energie ein weiteres Auftreffereignissignal zur Verfügung zu stellen,
- eine Logikschaltung (2) enthält, die dazu eingerichtet ist, das erste Auftreffereignissignal mit dem wenigstens einen weiteren Auftreffereignissignal zu vergleichen und abhängig von einem Ergebnis des Vergleichs ein Koinzidenzsignal zur Verfügung zu stellen,
- einen Koinzidenzzähler (3) enthält, der dazu eingerichtet ist, abhängig von dem Koinzidenzsignal einen Koinzidenzzählerstand des Koinzidenzzählers (3) zu inkrementieren, und
- für jedes weitere Detektorpixel (12) des wenigstens einen weiteren Detektorpixels (12) eine Schalteinheit (4) enthält, die dazu eingerichtet ist, das jeweilige weitere Auftreffereignissignal von der Logikschaltung (2) zu trennen.

2. Elektronische Schaltung (10) nach Anspruch 1, wobei die erste Erfassungseinheit (1) einen Komparator (5) enthält und/oder die wenigstens eine weitere Erfassungseinheit (1) jeweils einen weiteren Komparator (5) enthält.

3. Elektronische Schaltung (10) nach einem der vorherigen Ansprüche, wobei
- jedes weitere Detektorpixel (12) des wenigstens einen weiteren Detektorpixels (12) an das Detektorpixel (11) grenzt, oder
- jedes weitere Detektorpixel (12) des wenigstens einen weiteren Detektorpixels (12) in einer vordefinierten Nachbarschaft des Detektorpixels (11) liegt.

4. Elektronische Schaltung (10) nach einem der vorherigen Ansprüche, aufweisend einen Steuerschaltkreis (6), der dazu eingerichtet ist, einen Schaltzustand der wenigstens einen Schalteinheit (4) zu steuern.

5. Elektronische Schaltung (10) nach einem der vorherigen Ansprüche, wobei die jeweilige Schalteinheit (4) einen Multiplexer (7) enthält, der dazu eingerichtet ist, der Logikschaltung (2) alternativ zu dem jeweiligen weiteren Auftreffereignissignal ein Alternativsignal (8) zur Verfügung zu stellen.

6. Elektronische Schaltung (10) nach einem der Ansprüche 1 bis 4, wobei die jeweilige Schalteinheit (4) dazu eingerichtet ist, der Logikschaltung (2) alternativ zu dem jeweiligen weiteren Auftreffereignissignal ein Alternativsignal (8) oder ein konstantes Bezugspotential zur Verfügung zu stellen.

7. Elektronische Schaltung (10) nach einem der vorherigen Ansprüche, wobei die elektronische Schaltung (10)
- eine zweite Erfassungseinheit (1') enthält, die dazu eingerichtet ist, abhängig von der mittels des Detektorpixels (11) erfassten Energie ein zweites Auftreffereignissignal zur Verfügung zu stellen, und
- einen Auftreffereigniszähler (9) enthält, der dazu eingerichtet ist, abhängig von dem zweiten Auftreffereignissignal einen Zählerstand des Auftreffereigniszählers (9) zu inkrementieren.

8. Elektronische Schaltung (10) nach einem der vorhergehenden Ansprüche, wobei die elektronische Schaltung (10) als ASIC oder als integrierte Schaltung ausgeführt ist.

9. Elektronische Schaltung (10) nach den einem der vorherigen Ansprüche, wobei die elektronische Schaltung (10) eine Umschalteinheit (13) enthält, welche dazu eingerichtet ist, dem Koinzidenzzähler (3) wahlweise das erste Auftreffereignissignal oder das Koinzidenzsignal zur Verfügung stellen.

10. Elektronische Schaltung (10) nach einem der vorherigen Ansprüche, wobei die Logikschaltung (2)
- ein ODER-Gatter (15) enthält, das eingangsseitig mit dem wenigstens einen weiteren Auftreffereignissignal verbunden ist und das dazu eingerichtet ist, an einem Ausgang des ODER-Gatters (15) ein Nachbarereignissignal zur Verfügung zu stellen, und
- ein UND-Gatter (16) enthält, das eingangsseitig mit dem ersten Auftreffereignissignal und dem Nachbarereignissignal verbunden ist und das dazu eingerichtet ist, an einem Ausgang des UND-Gatters (16) das Koinzidenzsignal zur Verfügung zu stellen.

11. Photonenzählender Röntgendetektor für ein CT-System, aufweisend eine elektronische Schaltung (10) nach einem der vorherigen Ansprüche.

12. CT-System aufweisend
- eine Röntgenröhre zum Aussenden von Röntgenphotonen;
- einen photonenzählenden Röntgendetektor nach Anspruch 11, und
- ein Datenverarbeitungssystem, das dazu eingerichtet ist, CT-Bilddaten abhängig von dem Koinzidenzzählerstand des Koinzidenzzählers (3) zu erzeugen.

13. Verfahren zum Erfassen von Koinzidenzereignissen eines CT-Systems, welches einen photonenzählenden Röntgendetektor aufweist, der ein Detektorpixelarray enthält, bei dem
- abhängig von einer mittels eines Detektorpixels (11) des Detektorpixelarrays erfassten Energie ein erstes Auftreffereignissignal zur Verfügung gestellt wird,
- für jedes weitere Detektorpixel (12) wenigstens eines weiteren Detektorpixels (12) des Detektorpixelarrays abhängig von einer mittels des jeweiligen weiteren Detektorpixels (12) erfassten Energie ein weiteres Auftreffereignissignal zur Verfügung gestellt wird,
- das erste Auftreffereignissignal mittels einer Logikschaltung (2) mit dem wenigstens einen weiteren Auftreffereignissignal verglichen wird und abhängig von einem Ergebnis des Vergleichs ein Koinzidenzsignal zur Verfügung gestellt wird und wobei abhängig von dem Koinzidenzsignal ein Koinzidenzzählerstand inkrementiert wird,
- in einem ersten Betriebsmodus des CT-Systems der Logikschaltung (2) jedes des wenigstens einen weiteren Auftreffereignissignals eingangsseitig zur Verfügung gestellt wird, und
- in einem zweiten Betriebsmodus des CT-Systems zumindest ein weiteres Auftreffereignissignal des wenigstens einen weiteren Auftreffereignissignals von der Logikschaltung (2) getrennt wird.

14. Verfahren nach Anspruch 13, wobei
- am Ende eines vorgegebenen Auslesezeitraums ein finaler Koinzidenzzählerstand des Koinzidenzzählers (3) bestimmt wird, und
- abhängig von dem finalen Koinzidenzzählerstand CT-Bilddaten erzeugt werden.

15. Verfahren nach Anspruch 14, wobei
- abhängig von der mittels des Detektorpixels (11) erfassten Energie eine Anzahl von Auftreffereignissen während des Auslesezeitraums bestimmt wird, und
- die CT-Bilddaten abhängig von dem finalen Koinzidenzzählerstand und der Anzahl von Auftreffereignissen während des Auslesezeitraums erzeugt werden.
